# EUROPEAN PATENT SPECIFICATION

(11) **EP 3 614 999 B1**
(45) Date of publication and mention of the grant of the patent: **13.07.2022**
(21) Application number: 18724083.3
(22) Date of filing: 26.04.2018
(51) Int. Cl.: A61K 8/49, A61Q 5/04, C07D 213/74, C07D 213/32

(54) **ODORLESS THIOLS FOR PERMANENT WAVING, STRAIGHTENING AND DEPILATORY APPLICATIONS**
GERUCHLOSE THIOLE FÜR DAUERHAFTE WELLUNG, GLÄTTUNG UND DEPILATIONSANWENDUNGEN
THIOLS INODORES POUR DES APPLICATIONS D'ONDULATION PERMANENTE, DE LISSAGE ET D'ÉPILATION

(30) Priority: 27.04.2017 US 201715498566
(43) Date of publication of application: 04.03.2020
(73) Proprietor: The Procter & Gamble Company, Cincinnati, OH 45202 (US)
(72) Inventor: ZHANG, Guiru, Lebanon Ohio 45036 (US); DAVIS, Stephanie, Lee, Cincinnati Ohio 45202 (US); MURPHY, Bryan, Patrick, Loveland Ohio 45140 (US); SALLOUM, David, Cincinnati Ohio 45202 (US)
(74) Representative: P&G Patent Germany
(86) International application number: PCT/US2018/029513
(87) International publication number: WO 2018/200772

(56) References cited:
- RU-C1- 2 032 670
- US-B1- 6 302 119
- US-B1- 6 384 063
- DATABASE REGISTRY [Online] CHEMICAL ABSTRACTS SERVICE, COLUMBUS, OHIO, US; 23 September 2004 (2004-09-23), "1-methyl-3-(3-mercaptoproyl) pyridinium", XP002783035, Database accession no. 749833-75-8
- DATABASE CAPLUS [Online] CHEMICAL ABSTRACTS SERVICE, COLUMBUS, OHIO, US; 1 April 1959 (1959-04-01), XP002783036, Database accession no. 1959:122146 & K. C. KENNARD ET AL: "Dithiocarbamates. I. Quaternary Ammonium Dithiocarbamates", JOURNAL OF ORGANIC CHEMISTRY, vol. 24, no. 4, 1 April 1959 (1959-04-01), pages 464-469, XP055492424, US ISSN: 0022-3263, DOI: 10.1021/jo01086a007
- Robert Rippel: "Mercaptoacetic Acid and Derivatives" In: "Ullmann's Encyclopedia of Industrial Chemistry", 15 June 2000 (2000-06-15), Wiley-VCH, Weinheim, XP055492468, ISBN: 978-3-527-30673-2 pages 555-558, DOI: 10.1002/14356007.a16_265,

## Description

### FIELD OF THE INVENTION

Described herein is the design and synthesis of a class of odorless thiols, particularly odorless thiols that are compounds that have in their molecule (a) a pyridine quaternary ammonium salt and (b) an aliphatic thiol functional group or a protected aliphatic thiol functional group.

### BACKGROUND OF THE INVENTION

Reducing agents or reductants are substances that take part in chemical reactions by causing other substances to be reduced. During this chemical transformation, the reducing agent is oxidized by giving electrons to the reduced substance. There are various processes that utilize reducing agents. These can include styling hair treatments such as permanent shaping, waving, and straightening as well as depilation, which involves removal of unwanted hair. Wool is also occasionally treated with reducing agents to improve elongation, elasticity and to facilitate its dyeing process.

The permanent shaping and/or alteration of the curvature of keratinous fibers, in particular human hair, by the application of ammonium thioglycolate (perm salt), or sodium thiogylcolate, is known. In order to provide the consumer with the desired waved or straightened hair, an alkaline solution of perm salt is often utilized as the first step of the process. Thioglycolate reducing agent enters the hair shaft and reduces the -S-S- disulfide bond of hair proteins. This step allows hair fibers to relax from their original shape. After hair is set to its desired new wavy patterns or straightened, a formulation containing hydrogen peroxide, an oxidizing agent, is applied to reform the disulfide bonds and oxidize any remaining unreacted thioglycolate.

Permanent waving and straightening products are typically sold in the form of kits containing a relaxing component, e.g., an alkaline solution of a reducing agent, and an oxidizing component, e.g., a hydrogen peroxide solution. In use, the relaxing component is first applied to hair to either create wave patterns or straighten hair by breaking the disulfide bonds in keratin proteins. After waiting a certain amount of time for the perm salt to do the chemistry, the hair is rinsed. Then, an oxidizing cream or gel is applied to reform a good portion of the disulfide bonds and thus fix the new curvature or newly straightened hair.

Chemical depilation is typically done using compositions having a reducing agent and high pH. Thioglycolate reducing agent is typically used to break the disulfide bond. Depilatory compositions are typically sold as gels, mousses, creams, lotions or peelable films. The composition is typically applied directly on skin where undesirable hair needs to be removed. After a certain amount of time, typically 1-10 minutes, the composition is removed by wiping it off the bodily surface. Next, the skin is rinsed to complete the process. The same technology can be used in a shaving cream to help soften facial hair, which makes subsequent shaving go easier and smoother.

Since Arnold F. Willatt first invented the cold perm using thioglycolate in 1938, many attempts have been made by the perming industry to reduce or eliminate the offensive odor associated with the otherwise very effective treatment. Masking with fragrance is typically attempted for all of the compositions that contain thioglycolate and derivatives. However, the offensive thioglycolate odor cannot be completely masked, especially post treatment. Modifying the thioglycolate to reduce its offensive odor has also been tried. Thiols with higher molecular weight are generally less volatile, thus less of it reaches the olfactory cells. However, it would also be more difficult for a bigger molecule to penetrate hair to remain effective. In the case of higher molecular weight thiols, there is also the difficulty of formulating a less polar ingredient in aqueous chassis. One of the ways to address this polarity issue is to include polar functional groups to the molecule in order to make it more water soluble. However, one of the adverse effects for high polarity is lower penetration of the material into the hair fibers. With lower penetration, the efficiency of the thiol is reduced.

Thiols carrying an aliphatic quaternary ammonium cation have also been explored to minimize the sulfurous stench. They are stable at acidic and neutral conditions. Under in-use conditions at high pH, however, these thiols degrade to generate a tertiary amine and a cyclic sulfide, which defeats the purpose of an odor free technology.

There have also been attempts to use cross-linking reagents without the thiol alcohol functionality to replace thioglycolate. The fact that Brazilian perm uses up to 10 wt.% formaldehyde to relax curly hair highlights the desperation of the perming and straightening industry to disassociate itself from the pungent stench of thioglycolate salts. However, the styling benefits of such treatments are not as durable as the reduction-oxidation process achieves. In addition, there are toxicity concerns about the materials used.

US 6,302,119 B1 is related to compositions and methods for reducing odors associated with permanent hair waving by contacting the hair with a disulfide before, during, or after application of a sulfur-based reducing agent.

Robert Rippel: "Mercaptoacetic Acid and Derivative" in: "Ullmann's Encyclopedia of Industrial Chemistry", 15 June 2000, Wiley-VCH, Weinheim refers to mercaptoacetic acid and its uses.

Accordingly, there is a need for a solution to using thioglycolates to create desirable hair styles as well as for depilatory compositions without the undesirable in-use experience, including odor.

### SUMMARY OF THE INVENTION

An odorless thiol compound is provided and comprises

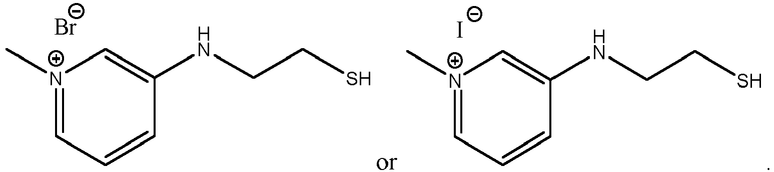

### DETAILED DESCRIPTION OF THE INVENTION

While the specification concludes with claims which particularly point out and distinctly claim the invention, it is believed the present invention will be better understood from the following description.

All percentages, parts and ratios are based upon the total weight of the compositions of the present invention, unless otherwise specified. All such weights as they pertain to listed ingredients are based on the active level and, therefore, do not include solvents or by-products that may be included in commercially available materials, unless otherwise specified. When more than one composition is used during a treatment, as in mixing of the components of a typical perming/straightening product, the total weight to be considered is the total weight of all the compositions applied on the hair simultaneously (i.e. the weight found "on head") unless otherwise specified. The term "weight percent" may be denoted as "wt. %" herein.

As used herein, the term "hair" to be treated may be "living" i.e. on a living body or may be "non-living" i.e. in a wig, hairpiece or other aggregation of non-living keratinous fibers. Mammalian, particularly human, hair is preferred. However, wool, fur, and other keratin containing fibers are suitable substrates for the compositions according to the present invention.

The hair permanent waving or straightening compositions can comprise one or more cationic thiols.

The inventors have surprisingly found that the use of thiols, which have in their molecule (a) a pyridine quaternary ammonium salt and (b) an aliphatic thiol functional group or a protected aliphatic thiol functional group, achieve reduction of the S-S bond of hair proteins without having an objectionable odor.

The disulfide compounds as disclosed herein can be perceived as protected thiols. This is because they can be readily converted to unprotected odorless thiols before their actual use in permanent hair styling or depilatory treatment, by a chemical reaction between another reducing agent with the disulfide compound.

Odorless thiols are described in detail hereinafter. The odorless thiols are a chemical class of reducing agents which are selected from the group containing compounds that can be represented by the following chemical structures:

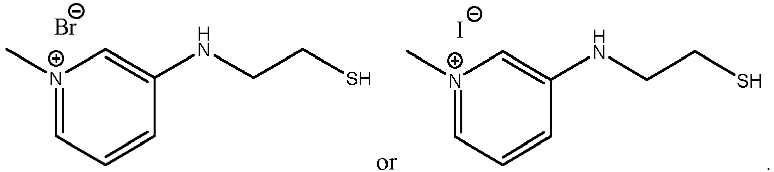

### I. Odorless Thiols

Described herein is a general method to derive odorless thiol reducing agents according to the compounds defined herein. Also described herein are methods and compositions for the reshaping of keratin fibers comprising at least one odorless thiol according to the compounds defined herein. Also described herein are methods comprising applying compositions comprising such reducing agents to the keratin fiber for a period of time, followed by an oxidizing agent, to shape the hair and develop the desirable hair style. Also described herein are methods comprising applying compositions comprising such reducing agents to bodily surfaces for a period of time sufficient to remove unwanted hair from the bodily surface.

It is understood that numerous potentially and actually tautomeric compounds are involved. It is to be understood that when this development refers to a particular structure, all of the reasonable additional tautomeric structures are included. In the art, tautomeric structures are frequently represented by one single structure and the disclosure herein follows this general practice.

The typical molecular structure of the reducing agents involves odorless thiols. These odorless thiols can be compounds that have in their molecule (a) a pyridine quaternary ammonium salt and (b) an aliphatic thiol functional group or a protected aliphatic thiol functional group. These compounds do not have an offensive odor, in contrast to the traditionally used thioglycolates, because the presence of the aromatic quaternary ammonium salt in their molecule imparts extremely low volatility. The low volatility contributes to a very low concentration of such compounds in the air and, as a result, the compounds will not be detected by olfactory cells during and after the treatment. The reducing agents are effective reducing agents for use in typical permanent waving, straightening and depilatory applications.

There are additional benefits associated with using odorless thiols under in-use conditions. Hair deposition/penetration can be greatly enhanced compared to thioglycolic acid (TGA) and its salts, which is anionic under in-use conditions. Thus, the reduction efficiency of the odorless thiols is increased. Penetration into live skin cells, however, is reduced as the cell membrane, a lipid bilayer, would only allow a certain cation and anion to go through the ion channels and keep most other salts out. This may result in less skin-irritating treatments.

Previous attempts to link a thiol to an aliphatic quaternary ammonium cation could not deliver an odorless technology at high pH, which is required for the deprotonation of the thiol to make it nucleophilic enough to break the hair protein disulfide bond efficiently. Such aliphatic quaternary ammonium salts can be stable and odorless under acidic and neutral conditions. At high pH, however, compound (III) below can be deprotonated to yield an unstable intermediate (IV), which can undergo a decomposition reaction to produce a tertiary amine (V) and a cyclic sulfide (VI), which can both carry unpleasant odors. The conversion rate of the degradation does not have to be high before the olfactory cells pick up the odor.

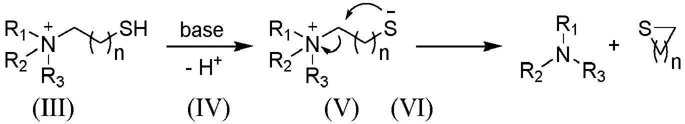

Similar to the above case, not all thiols linked to an aromatic cation are stable, either. Compound (VII) can be stable under acidic and neutral conditions. Under basic conditions, however, it can undergo a degradation reaction through intermediate (VIII) to provide (IX) and thiirane, which can make it an unsuitable candidate for the odor free technology.

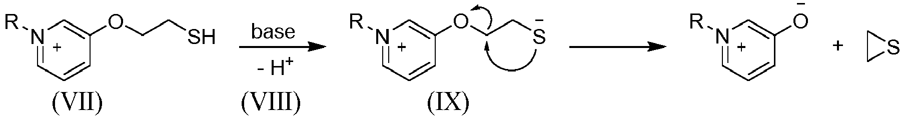

The odorless thiols reducing agents described herein differ from previous approaches in terms of high pH stability and undetectable level of odor. The odorless thiols have in their molecule an aromatic quaternary ammonium salt which contributes to an extremely stable compound at high pH. They are also efficient in breaking the disulfide bonds in keratin fibers. Some members of this class of compounds are three times more efficient than thioglycolate salts. Thus, in these cases substantially equal performance in perming can be achieved with 1/3 molar concentration of the active ingredient compared to the typically used ammonium thioglycolate salt. In addition, the reducing agents disclosed herein have high water solubility as they are organic salts, which makes them easy to formulate in an aqueous chassis. Finally, the reducing agents disclosed herein can pass the odor test in formulation and during on hair applications as stench free.

An example of a subclass of odorless thiols is represented by the following structure:

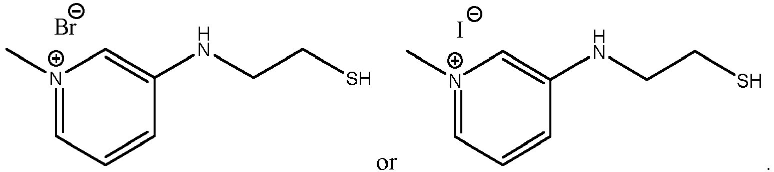

### PROTECTING GROUPS FOR THIOLS

The odorless thiols described herein can include thiols which have been functionalized with a protective group. A protecting group is a chemical moiety that has been reacted with a functional group of interest (thiol in this case). Such protection is encountered when functional groups are unstable or reactive under certain conditions. Typically, the protected functional group can then be deprotected with a simple transformation to make it again reactive so it can perform a desired function. Non-limiting examples of protecting groups for thiols can include acetyl group, tetrahydropyranyl group, a p-methylbenzyl group, a methoxymethyl group or a -(=NH₂⁺)NH₂ group. Commonly used protective groups can include trimethylacetamidomethyl, acetamidomethyl group and others. The compounds can also be disulfide compounds, which can be perceived as protected thiols. This is because these compounds can be readily converted to unprotected odorless thiols before their actual use in permanent hair styling or depilatory treatment, by a chemical reaction between another reducing agent with the disulfide compound.

### COUNTERIONS

The odorless thiols involve permanent quaternary ammonium salts.

### EXAMPLES

### METHOD OF PREPARATION OF EXAMPLES OF ODORLESS THIOLS

### A. Synthesis of 3-(mercaptomethyl)-1-methylpyridin-1-ium trifluoroacetate (not within the scope of the invention)

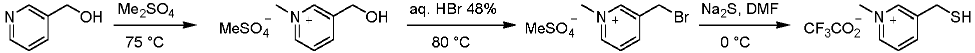

A scintillation vial equipped with silicone septum containing 2.00 g of pyridin-3-ylmethanol in 2.43 g dimethylsulfate is cooled in ice-water bath to 0 °C. The reaction mixture is then sealed with screw cap and allowed to warm up to room temperature and above while magnetically stirred. After the temperature rise ceases and the temperature of the reaction mixture drops back to ambient temperature, the reaction mixture is heated to 75 °C in an oil bath. The reaction mixture is kept at 75 °C for 1 hour while magnetically stirred. The resulting thick oil is purified using automated flash column chromatography on silica with dichloromethane and methanol as mobile phase to provide 4.10 g (95% yield) of 3-(hydroxymethyl)-1-methylpyridin-1-ium methylsulfate as a thick colorless oil.

Into a 500 mL round bottom flask, which initially contains 4.10 g 3-(hydroxymethyl)-1-methylpyridin-1-ium methylsulfate, is added a quantity of 150 mL of aqueous HBr (48 weight%). The reaction mixture is then heated to 80 °C while magnetically stirred. Once Liquid Chromatography Mass Spectrometry (LCMS) confirms the complete conversion of the alcohol to the corresponding bromide, the excess aqueous HBr is then removed using vacuum. The residual reddish brown oil is purified using automated flash column chromatography on silica with dichloromethane and methanol as mobile phase to provide 4.31 g (83% yield) of 3-(bromomethyl)-1-methylpyridin-1-ium methylsulfate as a thick amber colored oil.

Into a 100 mL evaporating flask, which initially contains 1.00 g of 3-(bromomethyl)-1-methylpyridin-1-ium methylsulfate, is added 30 mL of anhydrous dimethylformamide (DMF). The solution is then cooled in an ice-water bath. Into the chilled solution is then added a quantity of 0.31 g of sodium sulfide. The reaction mixture is then allowed to warm up to room temperature gradually while being magnetically stirred. The color of the solution becomes dark as the reaction proceeds. Once HPLC confirms that the conversion of the bromide to corresponding mercapto compound has stalled, the reaction is then quenched with 6 mL of a 1 N HCl solution. The solvents are then removed using vacuum. The residual dark brown slurry is purified using automated flash column chromatography on C-18 reverse phase column with water and acetonitrile with 0.1% trifluoroacetic acid (TFA) as mobile phase to provide 0.56 g (60% yield) of 3-(mercaptomethyl)-1-methylpyridin-1-ium trifluoroacetate as white solid.

### B. Synthesis of 3-((2-mercaptoethyl)amino)-1-methylpyridin-1-ium trifluoroacetate (not within the scope of the invention)

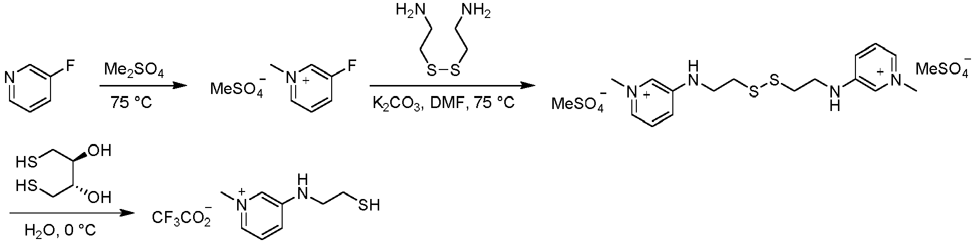

Into a scintillation vial equipped with a silicone septum, which initially contains 3.00 g of 3-fluoropyridine, is added 4.09 g of neat dimethylsulfate. The vial is then sealed and magnetically stirred. After the heat rise ceases and the temperature of the reaction mixture drops back to room temperature, the reaction mixture is heated to 75 °C on oil bath. The reaction mixture is kept at 75 °C for 1 hour while it is magnetically stirred. The resulting thick oil is purified using automated flash column chromatography on silica with dichloromethane and methanol as mobile phase to provide 6.41 g (93% yield) of 3-fluoro-1-methylpyridin-1-ium methylsulfate as a thick colorless oil.

Into a scintillation vial equipped with a silicone septum, which initially contains 1.50 g of 3-fluoro-1-methylpyridin-1-ium methylsulfate dissolved in 10 mL of DMF, is added 1.00 g of potassium carbonate and 0.47 g of 2,2'-dithiobis-ethanamine. The reaction mixture is then heated at 75 °C for 3 hours while magnetically stirred. Once the conversion is confirmed to be complete by Ultra Performance Liquid Chromatography (UPLC), potassium salts are filtered off and the DMF is removed using vacuum. The residual reaction mixture is then purified using automated flash column chromatography on silica with dichloromethane (DCM) and methanol as mobile phase to provide 1.55 g (91% yield) of 3,3'-((disulfanediylbis(ethane-2,1-diyl))bis(azanediyl))bis(1-methylpyridin-1-ium) methylsulfate as a pale yellowish solid.

Into a scintillation vial equipped with a silicone septum, which initially contains 1.00 g of 3,3'-((disulfanediylbis(ethane-2,1-diyl))bis(azanediyl))bis(1-methylpyridin-1-ium) methylsulfate dissolved in 10 mL of water and cooled to 0°C, is added a quantity of 0.33 g of DL-1,4-dithiothreitol. The reaction mixture is magnetically stirred in ice-water bath for 1 hour. Once the conversion is confirmed to be complete by UPLC, the reaction mixture is then purified using automated flash column chromatography on C-18 reverse phase column with methanol/H₂O with 0.1% TFA as mobile phase to provide 0.80 g (79% yield) of 3-((2-mercaptoethyl)amino)-1-methylpyridin-1-ium trifluoroacetate as a pale yellowish solid.

### C. Synthesis of 3-[bis(2-mercaptoethyl)amino]-1-methylpyridin-1-ium trifluoroacetate (not within the scope of the invention)

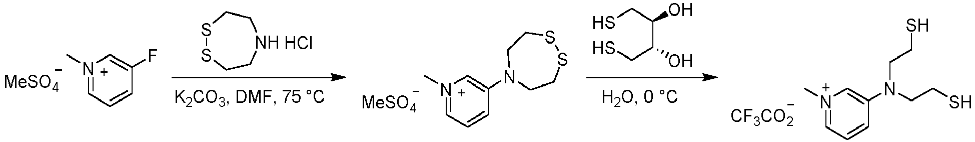

Into a scintillation vial equipped with a silicone septum, which initially contains 1.56 g of 3-fluoro-1-methylpyridin-1-ium methylsulfate dissolved in 10 mL of DMF, is added a quantity of 1.50 g of potassium carbonate and 1.00 g of 1,2,5-dithiazepane hydrochloride. The reaction mixture is then heated to 75 °C and is kept at this temperature for 2 hours while magnetically stirred. Once the conversion is confirmed to be complete by UPLC, the potassium salts are filtered off and the DMF is removed using vacuum. The residual reaction mixture is then purified using automated flash column chromatography on silica with DCM and methanol as mobile phase to provide 1.62 g (82% yield) of 3-(1,2,5-dithiazepan-5-yl)-1-methylpyridin-1-ium methylsulfate as a pale yellowish solid.

Into a scintillation vial equipped with a silicone septum, which initially contains 1.00 g of 3-(1,2,5-dithiazepan-5-yl)-1-methylpyridin-1-ium methylsulfate dissolved in 15 mL of water (15 mL) and cooled at 0°C, is added a quantity of 0.60 g of DL-1,4-dithiothreitol. The reaction mixture is magnetically stirred in ice-water bath for 1 hour. Once the conversion is confirmed to be complete by UPLC, the reaction mixture is then purified using automated flash column chromatography on C-18 reverse phase column with methanol/H₂O with 0.1% TFA as mobile phase to provide 0.74 g (73% yield) of 3-[bis(2-mercaptoethyl)amino]-1-methylpyridin-1-ium trifluoroacetate as a pale yellowish solid.

### D.Synthesis of 3-((2-((amino(indnio)methyl)thio)ethyl)amino)-1-methylpyridin-1-ium trifluoroacetate (not within the scope of the invention)

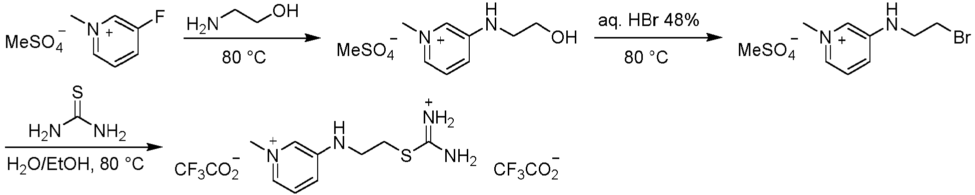

Into a scintillation vial equipped with a silicone septum, which initially contains 3.00 g of 3-fluoro-1-methylpyridin-1-ium methylsulfate and cooled to 0 °C, is added a quantity of 2.05 g of neat ethanolamine. The reaction mixture is then heated to 80 °C and is kept at this temperature for 3 hours while magnetically stirred. Once the conversion is confirmed to be complete using UPLC, the reaction mixture is then purified by automated flash column chromatography on silica with DCM and methanol as mobile phase to provide 3.41 g (96% yield) of 3-((2-hydroxyethyl)amino)-1-methylpyridin-1-ium methylsulfate (3.41 g) as a thick colorless oil.

Into a 500 mL round bottom flask, which initially contains 3.41 g of 3-((2-hydroxyethyl)amino)-1-methylpyridin-1-ium methylsulfate, is added a quantity of 150 mL of a aqueous HBr (48% solution). The reaction mixture is then heated to 80 °C while magnetically stirred. Once LCMS confirms the complete conversion of the alcohol to corresponding bromide, excess aqueous HBr is then removed using vacuum. The residual reddish brown oil is purified using automated flash column chromatography on silica with dichloromethane and methanol as mobile phase to provide 3.63 g (86% yield) of 3-((2-bromoethyl)amino)-1-methylpyridin-1-ium methylsulfate as a thick amber colored oil

Into a scintillation vial equipped with a silicone septum, which initially contains 1.00 g of 3-((2-bromoethyl)amino)-1-methylpyridin-1-ium methylsulfate dissolved in 20 mL water/ethanol (v/v = 1/1), is added a quantity of 0.28 g of thiourea. The reaction mixture is then heated in oil bath to 80 °C and is kept at this temperature for 1 hour while magnetically stirred. Once the conversion is confirmed to be complete by UPLC, the solvents are then removed using vacuum. The residual reaction mixture is then purified using automated flash column chromatography on C-18 reverse phase column with methanol/H₂O with 0.1% TFA as mobile phase to provide 1.23 g (92% yield) of 3-((2-((amino(iminio)methyl)thio)ethyl)amino)-1-methylpyridin-1-ium trifluoroacetate as a pale yellowish solid.

### E. Synthesis of 1,1'-(butane-1,4-diyl)bis(3-(mercaptomethyl)pyridin-1-ium) bromide (not within the scope of the invention)

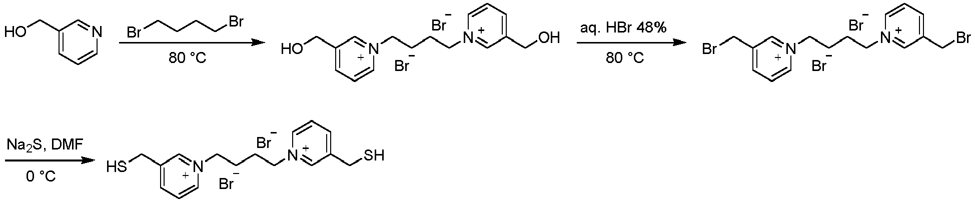

Into a scintillation vial equipped with a silicone septum, which initially contains 3.00 g of pyridin-3-ylmethanol, is added a quantity of 2.00 g of neat 1,4-dibromobutane. The reaction mixture is then sealed with screw cap and heated to 80 °C on oil bath. The reaction mixture is kept at this temperature overnight while it is magnetically stirred. The resulting solid is purified using automated flash column chromatography on silica with dichloromethane and methanol as mobile phase to provide 3.58 g (89% yield) of 1,1'-(butane-1,4-diyl)bis(3-(hydroxymethyl)pyridin-1-ium) bromide as a white solid.

Into a 250 mL round bottom flask, which initially contains 3.00 g of 1,1'-(butane-1,4-diyl)bis(3-(hydroxymethyl)pyridin-1-ium) bromide, is added a quantity of 100 mL of aqueous HBr (48% solution). The reaction mixture is then heated to 80 °C while magnetically stirred. Once LCMS confirms the complete conversion of the alcohol to the corresponding bromide, the excess aqueous HBr is then removed using vacuum. The residual reddish brown oil is purified using automated flash column chromatography on silica with dichloromethane and methanol as mobile phase to provide 3.29 g (85% yield) of 1,1'-(butane-1,4-diyl)bis(3-(bromomethyl)pyridin-1-ium) bromide as a thick amber colored solid.

Into a 100 mL evaporating flask, which initially contains 1.50 g of 1,1'-(butane-1,4-diyl)bis(3-(bromomethyl)pyridin-1-ium) bromide, is added a quantity of 30 mL of anhydrous DMF. The resulting solution is then cooled in an ice-water bath. Into the chilled solution is then added a quantity of 0.84 g of sodium sulfide. The reaction mixture is then allowed to warm up to room temperature gradually while being magnetically stirred. The color of the solution becomes dark as the reaction proceeds. Once HPLC confirms that the conversion of the bromide to the corresponding mercapto compound has stalled, the reaction is then quenched with 12 mL of a IN HBr solution. The solvents are then removed using vacuum. The residual dark brown slurry is purified using automated flash column chromatography on C-18 reverse phase column with water and acetonitrile as mobile phase to provide 0.72 g (58% yield) of 1,1'-(butane-1,4-diyl)bis(3-(mercaptomethyl)pyridin-1-ium) bromide as a white solid.

### METHOD OF TREATING HAIR

### Reducing Composition

Described herein is a method for treating hair comprising: (a) putting the hair in a desired shape; (b) before and/or after the hair is put in the desired shape, applying a reducing composition to the hair; (c) rinsing the reducing composition from the hair; (d) applying an oxidizing composition to the hair; and (e) rinsing the oxidizing composition from the hair with water.

The reducing composition can comprise from 2% to 20%, alternatively from 2% to 18%, alternative from 2.5% to 15%, alternatively from 5% to 13%, alternatively from 7% to 12%, and alternatively from 9% to 11% of an odorless thiol by weight of the reducing composition. The reducing composition can comprise only the odorless thiol. The odorless thiol can be 3-((2-mercaptoethyl)amino)-1-methylpyridin-1-ium iodide.

The reducing composition can comprise from 4% to 10%, alternatively from 5% to 7% of a buffering system, by weight of the reducing composition. The buffering system can comprise ammonium carbonate and/or ammonium hydroxide.

Suitable pH modifiers and/or buffering agents can include, but are not limited to: ammonia; alkanolamides (such as monoethanolamine, diethanolamine, triethanolamine, monopropanolamine, dipropanolamine, tripropanolamine, tripropanolamine, 2-amino-2-methyl-1-propanol, 2-amino-2-hydroxymethyl-1,3,-propandiol); guanidium salts; alkali metal and ammonium hydroxides and carbonates; and mixtures thereof.

Further pH modifiers and/or buffering agents can include, but are not limited to: sodium hydroxide; ammonium carbonate; acidulents (such as inorganic and inorganic acids including for example phosphoric acid, acetic acid, ascorbic acid, citric acid or tartaric acid, hydrochloric acid); and mixtures thereof.

The reducing composition can comprise from 50% to 93.5%, alternatively from 55% to 92.5%, alternatively from 60% to 92.5%, alternatively from 65% to 92.5%, alternatively from 70% to 90% of a solvent, by weight of the reducing composition.

The solvent can be selected from water, or a mixture of water and at least one organic solvent to dissolve the compounds that would not typically be sufficiently soluble in water.

Suitable organic solvents can include, but are not limited to: C1 to C4 lower alkanols (such as ethanol, propanol, isopropanol); aromatic alcohols (such as benzyl alcohol and phenoxyethanol); polyols and polyol ethers (such as carbitols, 2-butoxyethanol, propylene glycol, propylene glycol monomethyl ether, diethylene glycol monoethyl ether, monomethyl ether, hexylene glycol, glycerol, ethoxy glycol, butoxydiglycol, ethoxydiglycerol, dipropyleneglocol, polygylcerol); propylene carbonate; and mixtures thereof.

The solvent can also be selected from the group consisting of water, ethanol, propanol, isopropanol, glycerol, 1,2-propylene glycol, hexylene glycol, ethoxy diglycol, and mixtures thereof.

The reducing composition can have a pH of from 7 to 11, alternatively from 8 to 10.5, alternatively from 9 to 10.

The reducing composition can further comprise one or more optional ingredients selected from the group consisting of chelants, radical scavengers, thickeners, rheology modifiers, salt, carbonate ion sources, conditioning agents, surfactants, perfumes, and mixtures thereof.

The oxidizing composition can comprise from 0.5% to 12%, alternatively from 1% to 8%, and alternatively from 1% to 5% of an oxidizing agent, by weight of the oxidizing composition.

The oxidizing agent can be selected from water soluble peroxygen oxidizing agents. Watersoluble peroxygen oxidizing agents can include, but are not limited to, hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulfates and the like. These inorganic perhydrate salts may be incorporated as monohydrates, tetrahydrates etc. Alkyl and aryl peroxides, and or peroxidases, oxidases, and uricases and their substrates may also be used. Mixtures of two or more such oxidizing agents can also be used if desired. The oxidizing agents may be provided in aqueous solution or as a powder which is dissolved prior to use. The oxidizing agents may be selected from the group consisting of hydrogen peroxide, percarbonate, persulfates and combinations thereof.

A potential oxidizing agent for use herein is a source of peroxymonocarbonate ions formed in situ from a source of hydrogen peroxide and a hydrogen carbonate ion source. Moreover, this system can be particularly effective in combination with a source of ammonia or ammonium ions. Accordingly, any source of these peroxymonocarbonate ions may be used. Suitable sources for use herein can include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may be used both as an oxidizing agent and as a source of carbonate ions. Sources of carbonate ions, carbamate and hydrocarbonate ions can include sodium hydrogen carbonate, potassium hydrogen carbonate, ammonium carbamate, and mixtures thereof. The oxidizing composition is selected from the group consisting of potassium bromate, sodium bromate, sodium perborate, dehydroascorbic acid, hydrogen peroxide, urea peroxide, and mixtures thereof. The oxidizing agent can be hydrogen peroxide.

The oxidizing composition can comprise from 80% to 97%, alternatively from 85% to 97%, and alternatively from 90% to 95% of a solvent, by weight of the oxidizing composition.

The oxidizing composition can further comprise one or more optional ingredients selected from the group consisting of chelants, radical scavengers, thickeners, rheology modifiers, salt, carbonate ion sources, conditioning agents, surfactants, perfumes, and mixtures thereof.

The method can also be used for hair depilation/removal. Here, the pH of the reducing composition can be from 8 to 12, alternatively from 9 to 11.5, and alternatively 10.

The method of treating hair can first comprise separating the hair (which is washed and towel-dried) into multiple sections, and then these sections can be rolled onto curlers (optional for straightening). The curlers used for permanent waves can have a diameter of 5 mm to 13 mm, while the curlers used for straightening can have a diameter greater than 13 mm.

After the rolling on curlers is finished, the curlers can be thoroughly wetted down using the required quantity of the reducing composition, which can be from 60 g to 120 g.

The amount of time the permanent shaping composition stays on the hair can be from 1 minute to 30 minutes, alternatively from 15 minutes to 30 minutes. This action time can be shortened by adding heat via the use of a heat radiator or a hood dryer.

After the action time has elapsed that is sufficient for the permanent shaping, which is dependent upon hair quality, the pH value, the shaping effectiveness of the shaping agent, the desired level of change, as well as on the application temperature, the hair is then rinsed with water. Optionally, for straightening, the hair may be dried and then flattened with a heated device such as a flat iron to achieve desired shape.

Thereafter, the hair is oxidatively post-treated ("fixed"). The oxidizing composition can be used in a quantity of from 50 g to 200 g, alternatively from 80 g to 100 g, depending on hair thickness and length. The concentration of the oxidizing agent can vary depending on application time (normally 1 minute to 40 minutes, alternatively 5 minutes to 20 minutes) and application temperature (25 deg. C. to 50 deg. C.).

After an action period required for the fixing composition of from 3 minutes to 15 minutes, alternatively from 5 minutes to 10 minutes, the curlers are removed (if used). It can be advantageous if the hair is then finally shaped as desired and then dried.

### EXAMPLES

The following examples illustrate the compositions as described herein. The exemplified compositions may be prepared by conventional formulation and mixing techniques. It will be appreciated that other modifications of the compositions described herein within the skill of those in the art can be undertaken without departing from the spirit and scope of compositions described herein. All parts, percentages, and ratios herein are by weight unless otherwise specified. Some components may come from suppliers as dilute solutions. The amount stated reflects the weight percent of the active material, unless otherwise specified.

**Reducing Compositions**

| | % by weight |
|---|---|
| Composition A | |
| Odorless thiol¹ | 2.5-15 |
| Ammonium Hydroxide (aq. 28% active) | 4.5 |
| Water | qs to 100 |

| Composition B | |
|---|---|
| Odorless thiol¹ | 2.5-15 |
| Ammonium carbonate | 10 |
| Water | qs to 100 |

| Composition C | |
|---|---|
| Odorless thiol¹ | 2.5-15 |
| FlexiThix^{™ 2} | 5 |
| Phenoxyethanol | 0.3 |
| Sodium Benzoate | 0.2 |
| Disodium EDTA | 0.1 |
| Ammonium Hydroxide (aq. 28% active) | 4 |
| Water | qs to 100 |

| Composition D | |
|---|---|
| Odorless thiol¹ | 2.5-15 |
| Aculyn^{™} 46³ | 15.8 |
| Phenoxyethanol | 0.3 |
| Sodium Benzoate | 0.3 |
| Disodium EDTA | 0.1 |
| Ammonium Hydroxide (aq. 28% active) | 4 |
| Water | qs to 100 |

| Composition E | |
|---|---|
| Odorless thiol¹ | 2.5-15 |
| Plantaren^{®} 2000 N UP⁴ | 20 |
| Phenoxyethanol | 0.3 |
| Sodium Benzoate | 0.3 |
| Disodium EDTA | 0.1 |
| Ammonium Hydroxide (aq. 28% active) | 4 |
| Water | qs to 100 |

| Composition F | |
|---|---|
| Odorless thiol¹ | 2.5-15 |
| Foaming agent | 5 |
| Phenoxyethanol | 0.3 |
| Sodium Benzoate | 0.3 |
| Disodium EDTA | 0.1 |
| Ammonium Hydroxide (aq. 28% active) | 4 |
| Water | qs to 100.0% |

| | |
|---|---|
| ¹: The odorless thiol may be any odorless thiol described herein. ²: PVP polymer supplied by Ashland. ³: PEG-150/Stearyl/SMDI copolymer supplied by Rohm and Haas. ⁴: Chemical makeup supplied by BASF. | |

**Oxidizing Compositions**

| | % by weight |
|---|---|
| Composition G | |
| Hydrogen Peroxide | 0.5-12 |
| Water | qs to 100 |
| | |

| Composition H | |
|---|---|
| Hydrogen Peroxide | 3 |
| Cetyl/stearyl alcohol | 4 |
| Salicylic acid | 0.1 |
| Phosphoric acid | 0.09 |
| Etidronic acid | 0.01 |
| Fragrance | 0.4 |
| Water | qs to 100 |

| Composition I | |
|---|---|
| Hydrogen Peroxide | 2 |
| Salicylic acid | 0.1 |
| Disodium hydrogen phosphate | 0.2 |
| Phosphoric acid | 0.15 |
| Ethoxylated castor oil | 1 |
| Vinylpyrrolidone/styrene copolymer | 0.1 |
| Fragrance | 0.1 |
| Water | qs to 100 |

### DATA

### Mannequin Head Odor and Curl Expert Evaluation

Control perm: Omniperm^{™} with ammonium thioglycolate, available from Zotos Professional, including both the reducing and oxidizing steps.

**Test perm:**

| Reducing Composition | |
|---|---|
| 3-((2-mercaptoethyl)amino)-1-methylpyridin-1-ium iodide | 10 |
| Ammonium carbonate | 5 |
| Ammonium hydroxide | to pH = 9.6 |
| Water | qs to 100 |

| Oxidizing Composition | |
|---|---|
| Hydrogen Peroxide | 3 |
| Cetyl/stearyl alcohol | 4 |
| Salicylic acid | 0.1 |
| Phosphoric acid | 0.09 |
| Etidronic acid | 0.01 |
| Fragrance | 0.4 |
| Water | qs to 100 |

### Odor Assessments:

Treatment and expert sensory performed by licensed cosmetologist with 20+ years expertise in perms. All evaluations shown in graphs were done with hair in wet state, before drying. Sulfur odor was zero from the beginning with the test perm treatment. During treatment, the test perm had some ammonia odor from the ammonium hydroxide/carbonate buffer which is represented in Table 1, "Total Odor."

**Table 1.**

| **WET HAIR EVALUATIONS:** | Sulfur Odor | | Total Odor | |
|---|---|---|---|---|
| Scale: 0 (none) - 5 (most) | Control Perm | Test Perm | Control Perm | Test Perm |
| Hair processing | 5 | 0 | 5 | 3 |
| After neutralizing | 5 | 0 | 5 | 1 |
| After 1 wash | 4 | 0 | 4 | 0 |
| After 2 washes | 4 | 0 | 4 | 0 |
| After 3 washes | 3 | 0 | 3 | 0 |
| After 4 washes | 3 | 0 | 3 | 0 |
| After 5 washes | 3 | 0 | 3 | 0 |
| After 10 washes | 1 | 0 | 1 | 0 |
| After 15 washes | 1 | 0 | 1 | 0 |
| After 20 washes | 1 | 0 | 1 | 0 |

### Curl Retention Assessments:

Treatment and expert sensory performed by licensed cosmetologist with 20+ years expertise in perms. All evaluations shown in graphs were done with hair in dry state, after air drying. Photos are available for most time points as well.

**Table 2.**

| **AIR DRIED CURL EVALUATIONS:** | Curl Retention | |
|---|---|---|
| | Scale: 0 (none) - 5 (most) | |
| | Control Perm | Test Perm |
| After treatment | 5 | 5 |
| After 1 wash | 4 | 4 |
| After 2 washes | 4 | 4 |
| After 3 washes | 4 | 4 |
| After 4 washes | 4 | 4 |
| After 5 washes | 4 | 4 |
| After 10 washes | 4 | 4 |
| After 15 washes | 4 | 4 |
| After 20 washes | 4 | 4 |

### Conclusion:

3-((2-mercaptoethyl)amino)-1-methylpyridin-1-ium iodide test treatment provided equal curl retention (data in Table 2) through 20 washes to benchmark control perm but with significantly less odor than control (data in Table 1).

### Depilatory compositions

The depilatory composition can be applied to the skin for 2 or more minutes

A method of removing hair from the bodily surface, the method can comprise the steps of:
a. Applying the composition to the bodily surface where it is desired to remove hair;
b. Leaving the composition on the bodily surface for 1 to 15 minutes;
c. Removing the composition from the bodily surface.

| Ingredient | Weight % |
|---|---|
| Potassium thioglycolate | 5 |
| Xanthan gum | 0.5 |
| Urea | 8 |
| Glycerin | 1 |
| Sodium gluconate | 0.1 |
| CI0-30 Alkyl acrylate crosspolymer¹ | 1.5 |
| KOH | To adjust the pH to 11.5 (+/- 0.5) |
| Deionized water | QS |

| | |
|---|---|
| ¹ Ultrez 20 supplied by Lubrizol | |

## Claims

1. An odorless thiol compound comprising

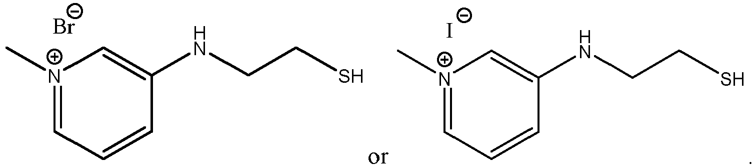

2. A permanent styling composition or a depilatory composition comprising from 1 wt.% to 20 wt.%, preferably from 3 wt.% to 12 wt.%, and more preferably from 5 wt.% to 12 wt.% of the odorless thiol according to Claim 1 by total weight of the composition.

3. Use of the odorless thiol compound having one of the structures of claim 1 for permanent styling or hair removal.

## Patentansprüche

1. Geruchlose Thiolverbindung, umfassend

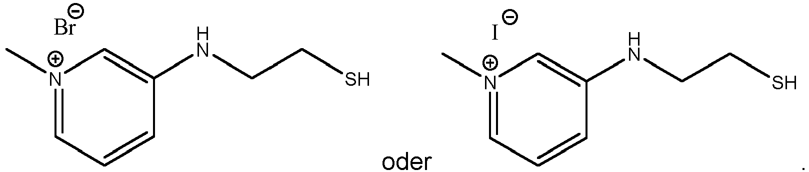

2. Zusammensetzung für ein dauerhaftes Styling oder Depilierzusammensetzung, zu 1 Gew.-% bis 20 Gew.-% der Zusammensetzung ausmacht, vorzugsweise zu 3 Gew.-% bis 12 Gew.-% der Zusammensetzung ausmacht und mehr bevorzugt zu 5 Gew.-% bis 12 Gew.-% der Zusammensetzung des geruchlosen Thiols nach Anspruch 1 definiert.

3. Verwendung der geruchlosen Thiolverbindung mit einer der Strukturen nach Anspruch 1 für ein dauerhaftes Styling oder zur Haarentfernung.

## Revendications

1. Composé de thiol sans odeur comprenant

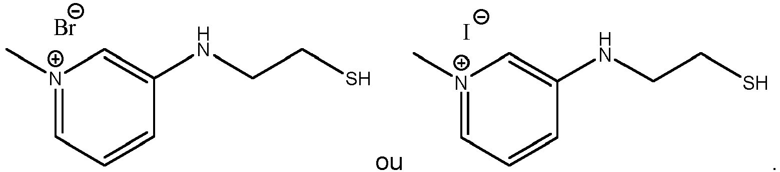

2. Composition de coiffage permanent ou composition dépilatoire comprenant de 1 % à 20 %, en poids de la composition, de préférence de 3 % à 12 %, en poids de la composition, et plus préférablement de 5 % à 12 %, en poids de la composition du thiol sans odeur selon la revendication 1.

3. Utilisation du composé thiol sans odeur ayant une des structures selon la revendication 1 pour un coiffage permanent ou une élimination des poils.
